(19) Europäisches Patentamt

European Patent Office

Office européen des brevets

(11) Publication number: **0 467 805 B1**

# EUROPEAN PATENT SPECIFICATION

(45) Date of publication of patent specification: **15.03.95**

(51) Int. Cl.6: **A61M 1/36**

(21) Application number: **91420251.0**

(22) Date of filing: **15.07.91**

(54) **Equipment for detecting the presence of a tube and/or the presence of fluid within it.**

(30) Priority: **17.07.90 IT 6755890**

(43) Date of publication of application:
**22.01.92 Bulletin 92/04**

(45) Publication of the grant of the patent:
**15.03.95 Bulletin 95/11**

(84) Designated Contracting States:
**AT BE CH DE DK ES FR GB GR IT LI LU NL SE**

(56) References cited:
**WO-A-82/00591**
**CH-A- 604 741**
**DE-A- 3 603 442**
**US-A- 3 812 482**

(73) Proprietor: **HOSPAL AG**
**Dornacherstrasse 8**
**CH-4008 Basel (CH)**

(72) Inventor: **Paolini, Francesco**
**Viale Della Repubblica No. 110**
**I-87100 Cosenza (IT)**
Inventor: **Rossi, Andrea**
**Via Cantone No. 92**
**I-46020 S.Giacomo Delle Segnate(Mantova)**
**(IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to equipment for detecting the presence of a tube and/or the presence of a fluid within it. In particular this invention is advantageously used in machines for the treatment of dialysis patients, especially during the stage in which the patient must be connected to the machine.

Known equipment of the abovementioned type comprises a light radiation emitter placed in such a way as to pass its radiation through a transparent tube in which blood circulates, and an optical sensor capable of receiving the light radiation emerging from the tube in order to provide a corresponding electrical signal to an electronic circuit. This equipment generally does not provide means to render the system insensitive to various disturbances.

Correct operation of such equipment is of extreme importance, because any error in correct determination of the state of the system may result in being particularly harmful to the patient. Errors may arise as a result of the deterioration and consequent malfunctioning of components in the electronic circuit, or ageing of the light emitter and/or optical sensor, or disturbance caused by optical interference phenomena.

U.S. patent 3 812 482 describes an equipment of the above mentioned type comprising a test circuit enabling to check a working condition of the equipment. However this test circuit is not able to differentiate a failure of the equipment caused by the optical component of it (light emitter, optical detector) from a failure caused by the electronic circuit associated with this optical component.

The object of the invention is to provide equipment for detecting the presence of a tube belonging to a circuit outside the body, and the presence of blood within it, which does not have the disadvantages of known equipment.

According to the invention, this object is accomplished by an equipment for detecting the presence of a tube and/or the presence of a fluid within it, comprising a light emitter directed towards the tube, an optical sensor capable of detecting the light leaving the tube in order to provide a corresponding electrical signal, a circuit for the processing of the signal from the optical sensor and a test circuit, characterized in that the test circuit is capable of being connected periodically to the processing circuit as a replacement for the light emitter and the sensor to generate at least one test signal SE' capable of checking that at least part of the equipment is functioning correctly, the test signal simulating the electrical signal SE generated in use by the optical sensor.

For a better understanding of the invention a preferred non-restrictive embodiment thereof will now be described with the help of the appended drawings, in which:

Figure 1 shows a partial block diagram of equipment for detecting the presence of a tube and/or the presence of blood within it according to the invention,

Figure 2 shows a median cross-section of an optical detector according to the equipment,

Figure 3 is a diagram of a series of threshold signals for the equipment in Figure 1,

Figures 4 and 5 are two flow diagrams illustrating two stages in the operation of the equipment.

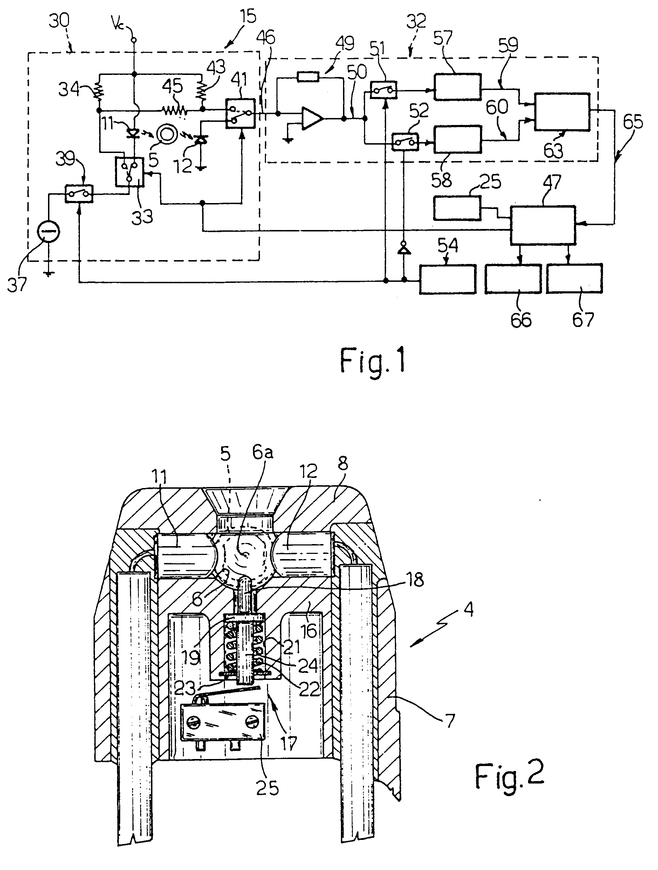

With reference to Figure 2, numeral 4 generically indicates the detector or sensor for haemoglobin in the blood of a patient forming part of measuring and/or monitoring equipment. This equipment is incorporated in a blood treatment unit, in particular a dialysis unit (not shown), provided with a tube 5, indicated by dashed lines in Figure 2, through which a patient's blood flows. This tube 5 is of a flexible transparent plastics material, for example polyvinyl chloride (PVC).

Detector 4 incorporates a holder consisting of a hollow body provided with a cylindrical seat 6, having an axis 6a, capable of receiving tube 5. Body 7 is covered by a removable lid 8 so that tube 5 may be inserted and removed.

Within body 7 and corresponding to seat 6 there are mounted in diametrically opposite positions an emitter 11 of light radiation and an optical receiver or sensor 12 for such radiation, which form the optical/electronic system of detector 4. Emitter 11 preferably consists of a light emitting diode, subsequently called LED (light emitting diode), which is connected to a control circuit. LED 11 is selected so as to generate light radiation having a wavelength close to the infra red, for example of the order of 800 nm, with a bandwidth of the order of 100 nm.

Optical sensor 12 (Figure 2) consists of a photodiode capable of receiving the light radiation emitted from LED 11 through tube 5. Because the light received by photodiode 12 varies with the concentration of haemoglobin in the blood in accordance with precise laws, the signal generated by photodiode 12 depends on the haemoglobin concentration of the blood. Photodiode 12 is connected to an electronic circuit 15 (Figure 1), which will be better seen below.

LED 11 and photodiode 12 (Figure 2) of this detector 4 project slightly with respect to the inner cylindrical surface of seat 6, so that the wall of tube 5 inserted therein is slightly compressed. This compression results in deformation of tube 5, which moulds itself to the geometrical shape of emitter 11 and receiver 12, preventing the astigmatic focussing which would arise from the cylindrical geometry of the tube filled with transparent liquid. This must be avoided because focussing of the beam would compensate for the absorption due to tube 5 and the contents thereof, making it impossible to distinguish between the situation where there was a tube full of transparent liquid and the situation in which a tube was absent.

An electromechanical sensor 17 capable of detecting the presence of tube 5 in seat 6 is mounted on a wall 16 of body 7 parallel to lid 8. Sensor 17 is substantially in the plane of LED 11 and photodiode 12, at right angles thereto, and comprises a pin 18 mounted on a piston 19 which slides in a cylindrical seat 21. Piston 19 is normally held at the end of its travel in the direction of wall 16 by a compression spring 22 located between this and a washer 23.

A lower end 24 of pin 18 acts in association with microswitch 25 so that when tube 5 is housed in seat 6 it presses pin 18 against the force of spring 22, activating microswitch 25. This is connected to a logic control circuit 47 (Figure 1), to which is then provided a digital electrical signal SA = 1 indicating that tube 5 is present. If tube 5 is not present the signal provided to circuit 47 is SA = 0.

As is known, disturbances due to ambient light on sensor 12 adversely effect correct measurement of the light received through tube 5. Even if the zone in which photodiode 12 is housed is darkened by hermetically enclosing body 7 it is impossible to eliminate the infiltration of light totally. In fact this light propagates along tube 5, which acts substantially as a waveguide.

In order to eliminate this source of disturbance LED 11 is supplied with an alternating signal conveniently consisting of a square wave. In such a way a signal corresponding alternately to the emission from LED 11 plus the light transmitted by tube 5 and a signal corresponding to only the light transmitted by tube 5 is obtained at the outlet from photodiode 12.

Circuit 15 (Figure 1) comprises a first block 30 consisting of a signal generating unit and comprising the optical/electronic system formed by LED 11 and photodiode 12. Unit 30 sends a signal, which is a function of the haemoglobin in the blood, to a second unit 32 consisting of a unit for the processing of that signal.

In particular LED 11 has the anode connected to a constant voltage source Vc and the cathode connected to a first output of a two way electronic switch 33, which has a second output connected to voltage source Vc by means of an interposed resistance 34. Switch 33 also has an input which is connected to a voltage generator 37 by the interposition of an electronic switch 39.

Photodiode 12 has the cathode connected to a first input of an electronic switch 41 which has a second input. This is connected to voltage source Vc by the interposition of a resistance 43, and with the second output of switch 33 by the interposition of a resistance 45. Electronic switch 41 also has an output terminal 46 connected to the unit 32 for processing the optical/electronic signal. Electronic switches 33 and 41 are activated by a bistable circuit included in the logic control circuit 47. The bistable circuit governs the switching of switches 33 and 41 in a synchronous manner on the basis of its state. The bistable circuit periodically replaces LED 11 and photodiode 12, through switches 33 and 41, with a test circuit configuration which simulates their behaviour so as to test that electronic circuit 15 is operating correctly.

Unit 32 comprises an impedance separator circuit 49 having an input connected to terminal 46 and having an output 50 which is connected to the inputs of two electronic switches 51 and 52. These switches are activated in opposing phases by an oscillator circuit 54 and the signal at output 50 is sampled. Also oscillator circuit 54 activates electronics switch 39 in phase with one of switches 51 and 52.

Each switch 51, 52 has one output respectively connected to the input of two memory blocks 57 and 58, which store the signal sampled by switches 51 and 52. These blocks 57 and 58 have outputs connected respectively to a first 59 and a second 60 input to a differential amplifier 63 which emits an analog electrical signal SE and sends it to a measuring circuit (not shown) via an output 65.

In use electronic circuit 15 has two separate operating configurations, based on the position adopted by switches 33 and 41: a first measuring operating configuration in which the level of haemoglobin present in the blood is measured and a second test operating configuration in which the performance of the entire system is checked.

In the first configuration switch 33 has its input in communication with its first output and switch 41 has its output in communication with its first input, whereby LED diode 11 is fed with an alternating voltage (for example a square wave) obtained by the subdivision of voltage Vc effected by the switching of switch 39.

Thus LED diode 11 emits light pulses with a frequency equal to that of the signal generated by oscillator circuit 54. As a result photodiode 12 produces a signal formed by the alternation of two unit signals: a first signal which is a function of the light generated in the LED diode 11 and received via tube 5 plus the light transmitted by tube 5 arising from the exterior (ambient light, etc.), and a second signal which is a function of only the light transmitted by tube 5. This signal passes through impedance separator 49 and is sampled by switches 51 and 52 so that the first and the second unit signals are stored in memories 57 and 58.

These first and second unit signals are then passed to differential amplifier 63 which provides an SE signal at output 65 which is proportional to the difference between them. Thus differential amplifier 63, by subtracting the second unit signal from the first unit signal, obtains an SE signal which is a function of only the light generated by LED diode 11, and is therefore free from any noise originating from the exterior.

The SE signal leaving amplifier 63 is passed to the measuring unit (not shown) first mentioned, which is able to process this signal and control a series of analog or digital indicators in a known way so as to express the level of haemoglobin in the blood.

It is therefore clear that thanks to oscillator 54 processing circuit 32 modulates the excitation signal which is sent to LED 11 and subsequently demodulates the signal received from photodiode 12. In particular the modulation and the demodulation are of the synchronous type and offer better cancellation of the various disturbances which can affect the optical/electronic circuit.

In the second working configuration switch 33 has its input in communication with its second output, which is connected to potential Vc by means of the separator resistances represented by resistances 34, 45 and 43. In turn switch 41 has its output in communication with its second input, which is connected to potential Vc through resistances 34, 45 and 43 and with generator 37 via resistance 45 respectively. In this configuration LED diode 11 and photodiode 12 are both separated from circuit 15.

Resistances 34, 45 and 43 are dimensioned in such a way as to supply a first component of the test signal at the output of switch 41, when switch 39 is open, which is substantially of the same order of magnitude as the signal generated by sensor 12 as a result of the effect of the light radiation from LED diode 11.

Resistances 34, 45 and 43 are also dimensioned so as to apply to the outlet of switch 41, when switch 39 is closed and the voltage contribution from generator 37 adds to the potential Vc, a second component of the test signal which on average is of the same order of magnitude as the signal generated by photodiode 12 as a result of the ambient light and the signal generated by LED diode 11.

These first and second components of the test signal are obtained by subdividing the reference voltage Vc and the voltage of voltage generator 37 (which are constant) by separator rsistances 34, 45 and 43. These components are therefore also generally constant in time and are of a known theoretical value.

In this way a signal consisting of an alternation of the first and second components of the test signal is present at the output of switch 41.

These components of the signal pass through impedance separator 49 and are sampled by switches 51 and 52 so that the first and second components of the test signal are stored in memories 57 and 58 respectively.

These first and second components of the test signal are then passed in turn to differential amplifier 63 which provides a signal SE' at output 65 which is proportional to the difference between them. In this way differential amplifier 63 obtains a third signal by subtracting the second component from the first component, which constitutes the real test signal SE'.

A priori knowledge of the theoretical value or reference value $V_{ref}$ of signal SE' makes it possible to perform an electronic test on units 30 and 32. With this object the condition:

$$|SE' - V_{ref}| < \epsilon$$

is checked at all times. In fact any changes in the functioning of the components of these circuits will alter the value of signal SE'.

In accordance with another aspect of the invention circuit 47 is also capable of controlling an error indicator 66 and a control unit 67 for a series of alarms. Error indicator 66 is designed to indicate an anomalous condition in the equipment. Control unit 67 is enabled during certain stages of operation to allow the equipment to activate a set of alarms. These alarms may be activated by various safety detectors, for example a detector of air bubbles in the line of tube 5, a detector of the lack of physiological solution for infusion into the line, pressure measuring devices on the line, etc., thus indicating any irregularities in the dialysis treatment.

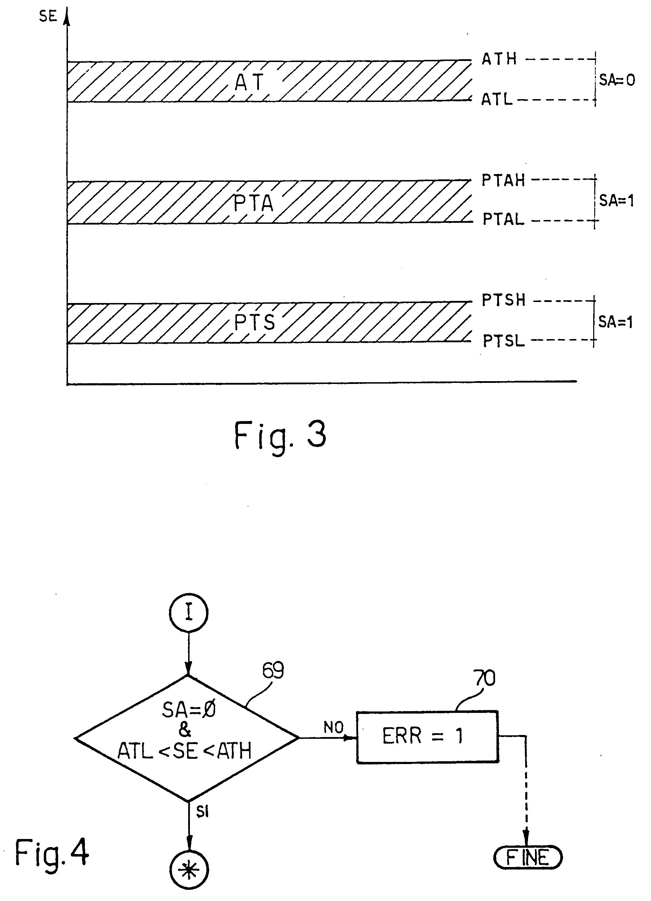

In the first configuration of the equipment described above circuit 47 is also able to compare SE signals received from amplifier 63 selectively with three pairs of threshold signals. A first pair ATH and ATL (Figure 3) of threshold signals represent the maximum and minimum limits of the expected value for signal SE emitted by amplifier 63 in a test performed with tube 5 absent. The two limits therefore represent the expected tolerance in this signal and define a field AT (absence of tube).

A second pair of threshold signals PTAH and PTAL represent the maximum and minimum limits of the expected value for a signal when tube 5 is present but empty of blood, or when tube 5 is empty or filled with physiological solution and is therefore not connected to the patient. The values for the threshold signals for this second pair define a field PTA (tube present blood absent) and are appreciably lower than the values for the first pair.

The third pair of threshold signals PTSH and PTSL represent the maximum and minimum limits of the expected value for a test signal when tube 5 is present and full of blood. The values of this third pair define a field PTS (tube present with blood) and are even lower than the values in the second pair.

As is known, when treating a patient with dialysis, the line incorporating tube 5 is first filled with physiological solution to start up the circulation. When the circulation has reached steady state conditions blood taken from a patient's vein is introduced into the line. Finally after treatment the line is emptied of blood by feeding in physiological solution.

The detection equipment described above functions in the following way.

When the equipment is switched on, before tube 5 is inserted in detector 4, microswitch 25 indicates that tube 5 is absent, generating the signal SA=0. Circuit 47 then performs an initial test 69 (Figure 4), consisting in checking the optical part of the circuit.

For this purpose signal condition SA=0 is checked and the SE obtained is compared to the threshold values ATL and ATH for checking the condition ATL<SE<ATH. If the result of one of the two checks is negative an error condition (ERR=1), represented by a block 70, is generated. Circuit 47 then enables error indicator 66 so that the operator should identify and eliminate the cause of the error before proceeding with treatment.

From what has been seen above it will be clear that sensor 17 indicating that the tube is present, including microswitch 25, can independently check whether tube 5 is present in detector 4 and any ageing in the optical electronic components (LED 11 and photodiode 12).

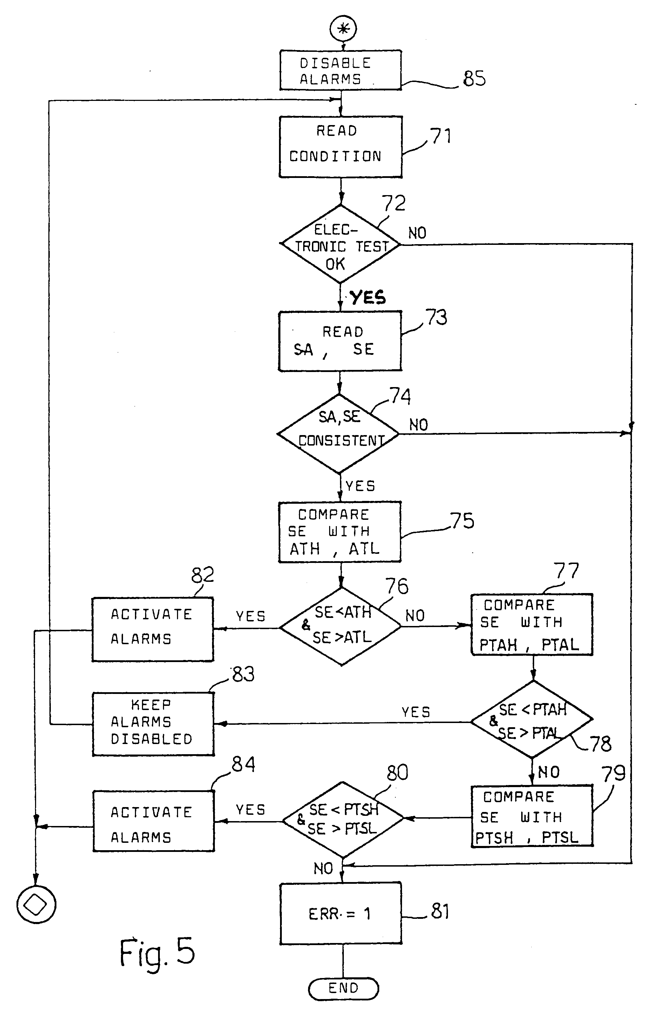

If however the result of the two checks represented by block 69 is positive, the operator can then place tube 5 in detector 4. Circuit 47 performs then the routine represented figure 5. This routine starts with an operation 85 whereby unit 67 controlling the alarms (see also Figure 1) is disabled. In fact at the start tube 5 inserted in detector 4 is empty or is filled with physiological solution. In this situation the alarms are not significant and would draw the attention of the operator without purpose. Operation 85 is followed by an operation 71 in which circuit 47 reads the condition of the electronic test, i.e. checks that a position signal for switches 33 and 41 is present within it.

A test 72 is then performed to check whether the result of the electronic test is satisfactory, i.e. whether signal SE' is within the expected limits. If the result of test of 72 is unsatisfactory an error indication is produced (block 81), as a result of which the routine stops immediately to allow the operator to act.

If on the other hand the result of tests 72 is satisfactory, the routine continues with an operation 73 in which the two signals SA and SE are read. A first test 74 then follows to check that the two signals SA and SE are consistent in accordance with the indications illustrated in Figure 3. If the result of test 74 is unsatisfactory, an error indication 81 is produced as in the previous case.

If on the other hand the result of test 74 is satisfactory a further operation 75 is performed in which the SE signal is compared with the two thresholds ATH and ATL. There now follows a test 76 for the conditions SE<ATH and SE>ATL. If the result of the test is satisfactory it means that tube 5 has not yet been inserted or has been removed. Circuit 47 then enables alarm control unit 67 (block 82) activating the alarms and the program passes to a suitable routine which is not indicated.

If instead the result of test 76 is unsatisfactory another operation 77 is performed in which the SE signal is compared with the pair of thresholds PTAH and PTAL. There then follows a check 78 for the conditions SE<PTAH and SE>PTAL. If the result of test 78 is satisfactory it means that tube 5 is inserted in detector 4, but that it is empty or still filled with physiological solution. As in this situation the alarms are not significant, unit 67 continues to be disabled (block 83) and the routine restarts from operation 71.

If the result of tests 78 is unsatisfactory a further comparison 79 is performed in which the signal is compared with the third pair of thresholds PTSH and PTSL. This is followed by a check 80 for the conditions SE<PTSH and SE>PTSL. If the result of this test 80 is satisfactory it means that tube 5 is inserted in detector 4 and full of blood, thus dialysis treatment is in progress. In this case alarm control unit 67 (block 84) is enabled. Unit 67 can then signal any malfunction in the course of treatment to the operator while the program passes into a corresponding routine which is not indicated. Obviously the alarms enabled by unit 67 with block 84 in the condition in which a tube is present with blood may be different from those enabled with block 82 in the condition in which the tube is absent.

Finally if test 80 has an unsatisfactory result it means that the SE signal does not fall in any of the three fields AT, PTA and PTS (Figure 3), which means that the equipment is faulty, as the measurements are not reliable. Error condition 81 is then displayed and the routine ends as in the previous cases of unsatisfactory results for tests 72 and 74.

From what has been seen above it is clear that the value of reference signal SE is compared with an interval of threshold values stored by control circuit 47 in each case and if the value does not lie within such an interval an error condition is displayed. It is also clear that the signal is always compared with a pair of thresholds consistent with the value of signal SA, and that the joint investigation of signals SA and SE makes it possible to evaluate any deterioration in any component of electronic circuit 15, in voltage source Vc and voltage generator 37, and therefore the state of efficiency of the entire equipment.

The switching of the bistable circuit included in circuit 47 makes it possible to have a series of electronic test cycles alternating with measurement cycles to provide a continuous check that the equipment is operating correctly. This switching may be performed manually, for example by pressing a "test" switch.

From what has been seen above the advantages of the equipment according to the invention over known equipment are obvious. In fact the test cycles may be performed in a very simple manner, and the test configuration enables the operator to check the operation of the entire equipment either before starting treatment or while treatment is in progress, and if a malfunction is revealed the patient's treatment may be restarted or suspended once it has already been started.

It is to be understood that various modifications and improvements may be made to the equipment described without going beyond the scope of the claims. For example electromechanical sensor 17, 25 may also be located outside device 4. Also the means of modulation and demodulation of the signal emitted by photodiode 12 may be different from those described, and in particular asynchronous. Also, instead of each pair of threshold values, a threshold value associated with a corresponding absolute permissible error value may be provided.

## Claims

1. Equipment for detecting the presence of a tube (5) and/or the presence of a fluid within it, comprising a light emitter (11) directed towards the tube (5), an optical sensor (12) capable of detecting the light leaving the tube (5) in order to provide a corresponding electrical signal, a circuit (32) for the processing of the signal from the optical sensor (12) and a test circuit, characterized in that the test circuit is capable of being connected periodically to the processing circuit (32) as a replacement for the light emitter (11) and the sensor (12) to generate at least one test signal (SE') capable of checking that at least part of the equipment is functioning correctly, the test signal simulating the electrical signal (SE) generated in use by the optical sensor (12).

2. Equipment according to claim 1, characterized in that the test circuit comprises at least one reference voltage source capable of providing the electrical signal (SE) and the test signal (SE').

3. Equipment according to claim 2, characterized in that the reference voltage source comprises a network of resistors (34, 45, 43) capable of being connected to a constant voltage source (Vc) to provide a first component for the test signal (SE').

4. Equipment according to claim 3, characterized in that the network of resistors (34, 45, 43) is also connected alternately to an auxiliary voltage source (37) to provide a second component for the test signal (SE').

5. Equipment according to claim 4, characterized in that the first and second components of the test signal (SE') alternate to simulate the behavior of the optical sensor (12) in response to the light emitter (11).

6. Equipment according to any one of claims 3 to 5, characterized in that it comprises comparator means (63) capable of generating the test signal (SE') from the difference between the first and the second components of the test signal (SE').

7. Equipment according to one of claims 1 to 6, characterized in that it comprises means (47) capable of comparing the value of the electrical signal (SE) with a series of stored values in order to detect any malfunction situation.

8. Equipment according to claim 7, characterized in that said comparison means are included in a control unit (47) capable of storing at least one threshold value (ATH, ATL) relative to the electrical signal (SE) in the condition in which the tube (5) is absent and at least one threshold value (PTAH, PTAL ; PTSH, PTSL) relative to the electrical signal (SE) in the condition in which the tube (5) is present, when blood is absent and present within the tube (5) respectively.

**9.** Equipment according to claim 8, characterized in that the control unit (47) is also capable of sensing a signal (SA) indicating that the tube (5) is present and to evaluate the consistency between the signal (SA) and the signal (SE) in order to evaluate the condition of efficiency of the light emitter (11) and the optical sensor (12).

**Patentansprüche**

**1.** Anlage zum Ermitteln der Anwesenheit eines Rohrs (5) und/oder der Anwesenheit eines Fluids in ihm, mit einem Lichtstrahler (11), der auf das Rohr (5) gerichtet ist, einem optischen Sensor (12), der dazu in der Lage ist, das Licht zu ermitteln, das das Rohr (5) verläßt, um ein entsprechendes elektrisches Signal zu erzeugen, einem Schaltkreis (32) zum Verarbeiten des Signals von dem optischen Sensor (12) und einem Testschaltkreis, dadurch gekennzeichnet, daß der Testschaltkreis dazu in der Lage ist, periodisch mit dem Verarbeitungsschaltkreis (32) als Ersatz für den Lichtstrahler (11) und den Sensor (12) verbunden zu werden, um zumindest ein Testsignal (SE') zu erzeugen, das dazu in der Lage ist, zu überprüfen, daß zumindest ein Teil der Anlage korrekt arbeitet, wobei das Testsignal das elektrische Signal (SE) simuliert, das bei der Verwendung durch den optischen Sensor (12) erzeugt wird.

**2.** Anlage nach Anspruch 1, dadurch gekennzeichnet, daß der Testschaltkreis zumindest eine Bezugsspannungsquelle umfaßt, die dazu in der Lage ist, das elektrische Signal (SE) und das Testsignal (SE') zu erzeugen.

**3.** Anlage nach Anspruch 2, dadurch gekennzeichnet, daß die Bezugsspannungsquelle ein Netzwerk aus Widerständen (34, 45, 43) umfaßt, das dazu in der Lage ist, an eine Konstantspannungsquelle (Vc) angeschlossen zu werden, um eine erste Komponente für das Testsignal (SE') zu erzeugen.

**4.** Anlage nach Anspruch 3, dadurch gekennzeichnet, daß das Netzwerk aus Widerständen (34, 45, 43) außerdem abwechselnd an eine Hilfsspannungsquelle (37) angeschlossen wird, um eine zweite Komponente für das Testsignal (SE') zu erzeugen.

**5.** Anlage nach Anspruch 4, dadurch gekennzeichnet, daß die ersten und zweiten Komponenten des Testsignals (SE') abwechseln, um das Verhalten des optischen Sensors (12) in Erwiderung auf den Lichtstrahler (11) zu simulieren.

**6.** Anlage nach einem der Ansprüche 3 bis 5, dadurch gekennzeichnet, daß sie eine Vergleichseinrichtung (63) umfaßt, die dazu in der Lage ist, das Testsignal (SE') aus der Differenz zwischen den ersten und zweiten Komponenten des Testsignals (SE') zu erzeugen.

**7.** Anlage nach einem der Ansprüche 1 bis 6, dadurch gekennzeichnet, daß sie eine Einrichtung (47) umfaßt, die dazu in der Lage ist, den Wert des elektrischen Signals (SE) mit einer Serie von gespeicherten Werten zu vergleichen, um eine Fehlfunktionssituation zu ermitteln.

**8.** Anlage nach Anspruch 7, dadurch gekennzeichnet, daß die Vergleichseinrichtung in einer Steuereinheit (47) enthalten ist, die dazu in der Lage ist, zumindest einen Schwellenwert (ATH, ATL) relativ zu dem elektrischen Signal (SE) unter der Bedingung abzuspeichern, in der das Rohr (5) nicht vorhanden ist, und zumindest einen Schwellenwert (PTAH, PTAL; PTSH, PTSL) relativ zu dem elektrischen Signal (SE) unter der Bedingung, in der das Rohr (5) vorhanden ist, wenn Blut in dem Rohr (5) jeweils nicht vorhanden und vorhanden ist.

**9.** Anlage nach Anspruch 8, dadurch gekennzeichnet, daß die Steuereinheit (47) außerdem dazu in der Lage ist, ein Signal (SA) zu erfühlen, das anzeigt, daß das Rohr (5) vorhanden ist, und die Übereinstimmung zwischen dem Signal (SA) und dem Signal (SE) zu ermitteln, um die Bedingung des Leistungsvermögens des Lichtstrahlers (11) und des optischen Sensors (12) zu ermitteln.

**Revendications**

**1.** Equipement pour détecter la présence d'un tube (5) et/ou la présence d'un fluide à l'intérieur du tube, comprenant un émetteur de lumière (11) orienté en direction du tube (5), un capteur optique (12) apte à détecter la lumière quittant le tube (5) pour fournir un signal électrique correspondant, un circuit (32) pour traiter le signal provenant du capteur optique (12), et un circuit de test, caractérisé en ce que le circuit de test est prévu pour être connecté périodiquement au circuit de traitement (32) comme substitut de l'émetteur de lumière (11) et du capteur (12) pour engendrer au moins un signal de test (SE') permettant de vérifier que au moins une partie de l'équipement fonctionne correctement, le signal de test simulant le signal électrique (SE) engendré en fonctionnement par le capteur optique (12).

2. Equipement selon la revendication 1, caractérisé en ce que le circuit de test comprend au moins une source de tension de référence apte à fournir le signal électrique (SE) et le signal de test (SE').

3. Equipement selon la revendication 2, caractérisé en ce que la source de tension de référence comprend un réseau de résistances (34, 45, 43) pouvant être connecté à une source de tension constante (Vc) pour fournir une première composante au signal de test (SE').

4. Equipement selon la revendication 3, caractérisé en ce que le réseau de résistances (34, 45, 43) est aussi connecté alternativement à une source de tension auxiliaire (37) pour fournir une seconde composante au signal de test (SE').

5. Equipement selon la revendication 4, caractérisé en ce que la première et la seconde composantes du signal de test (SE') alternent pour simuler le comportement du capteur optique (12) en réponse à l'émetteur de lumière (11).

6. Equipement selon une des revendications 3 à 5, caractérisé en ce qu'il comporte des moyens de comparaison (63) aptes à engendrer le signal de test (SE') à partir de la différence entre la première et la seconde composantes du signal de test (SE').

7. Equipement selon une des revendications 1 à 6, caractérisé en ce qu'il comporte des moyens (47) aptes à comparer la valeur du signal électrique (SE) avec une série de valeurs mémorisées pour détecter toute situation de disfonctionnement.

8. Equipement selon la revendication 7, caractérisé en ce que les moyens de comparaison sont inclus dans une unité de commande (47) apte à stocker au moins une valeur de seuil (ATH, ATL) relative au signal électrique (SE) dans la condition où le tube (5) est absent et au moins une valeur de seuil (PTAH, PTAL ; PTSH, PTSL) liée au signal électrique (SE) dans la condition où le tube (5) est présent, lorsque le sang est absent et est présent dans le tube (5) respectivement.

9. Equipement selon la revendication 8, caractérisé en ce que l'unité de commande (47) est aussi apte à détecter un signal (SA) indiquant que le tube (5) est présent et à évaluer la cohérence entre le signal (SA) et le signal (SE) dans le but d'évaluer le fonctionnement de l'émetteur de lumière (11) et du capteur optique (12).

Fig.1

Fig.2

Fig. 3

Fig.4

Fig. 5